# EUROPEAN PATENT SPECIFICATION

(11) **EP 1 322 348 B1**
(45) Date of publication and mention of the grant of the patent: **11.11.2009**
(21) Application number: 01970481.6
(22) Date of filing: 28.09.2001
(51) Int. Cl.: A61L 24/00, A61L 15/58

(54) **SKINFRIENDLY ADHESIVE WITH PH-LOWERING SUBSTANCE**
HAUTFREUNDLICHE KLEBSTOFFE MIT PH-WERT-SENKENDEN SUBSTANZEN
ADHESIF RESPECTUEUX DE LA PEAU POURVU D'UNE SUBSTANCE REDUISANT LE PH

(30) Priority: 02.10.2000 SE 0003537
(43) Date of publication of application: 02.07.2003
(73) Proprietor: Mölnlycke Health Care AB, 402 52 Göteborg (SE)
(72) Inventor: FABO, Tomas, S-435 41 Mölnlycke (SE)
(74) Representative: Hyltner, Jan-Olof
(86) International application number: PCT/SE2001/002101
(87) International publication number: WO 2002/028447

(56) References cited:
- EP-A1- 0 564 307
- WO-A1-96/05813
- WO-A1-97/34947

## Description

### TECHNICAL FIELD

The present invention relates to a skin-friendly adhesive and to a wound dressing which is provided with such an adhesive.

### BACKGROUND TO THE INVENTION

Healthy skin normally has a pH of between 4.0 and 6.0, which, according to expert opinion, is the most favourable environment for the skin. There are a number of different pH-dependent biological and biochemical mechanisms which contribute to the optimum pH for the skin being within this range.

It has been known for a long time that the "acid cloak" provides a direct protection against undesirable microorganisms, which do not flourish in the acid environment.

One of the most important functions of the skin is to constitute a two-way barrier which firstly regulates evaporation of water from the body and secondly prevents undesirable substances and particles from penetrating the skin from the outside. For example, the barrier protects against bacteria, fungi, viruses, allergens and toxic substances. The barrier also protects against proteolytic enzymes which can attack the skin when pus or faeces comes into contact with the skin. Research has shown that a naturally weakly acid pH of the skin round about a pH of 5 is a prerequisite for maintaining the normal barrier function of the skin. For example, it has been shown that using alkaline and neutral soaps damages the skin barrier much more than does using acid soaps. Various enzyme reactions in the stratum corneum which regulate the assembly of the sensitive lipid barrier are heavily dependent on the pH, which also suggests that it is important for the skin to maintain a naturally acid pH.

The barrier function of skin which surrounds wounds and skin which is affected by various skin diseases is often damaged. The skin barrier is frequently greatly impaired due to the underlying disease or damage. For example, the skin surrounding venous leg ulcers is extremely thin and sensitive. In certain cases, the barrier is impaired still further by the medical treatment. The skin can also be weakened by radiotherapy and cortisone treatment, which often have to be initiated in the case of patients with wounds. In addition, the skin is frequently stressed still further in connection with changes of dressing. In general, the adhesive on many commonly occurring self-adhesive dressings draws with it a layer of corneocytes when the dressings are detached from the skin. When dressings are changed repeatedly, the changes contribute to a weakening of the barrier still further. Another side-effect in connection with wound and skin care is eczema, and accordingly an impaired barrier, which can be obtained from ointments, plaster adhesives and topical medications. Another factor which has a negative effect on the health of the skin is occlusion, which has been shown to increase the pH of the skin and weaken its barrier. This effect is achieved when a tape or dressing is adhered to the skin, in connection with which the moisture content increases in the contact surface under the adhesive. After a few days, it can be seen that the skin is moist under the dressing and it is possible to measure a pH value which is increased and is often around 7. The lower the vapour permeability of the dressing or the tape, the more rapid and more pronounced does this effect become. After 3-4 weeks of occlusion, an inflammation normally arises as a result of the increased moisture content and the increased pH. The conclusion is that it is particularly important to maintain or, alternatively, restore a natural pH to the skin surrounding a wound and to skin which has been weakened in some other way.

The self-adhesive dressings and sticking plasters which are used today are provided with adhesives which are pH-neutral, i.e. they do not affect the pH of water which is in contact with the adhesive. None of these product types possesses adhesive which has been demonstrated to actively ensure the correct pH when the adhesive is in contact with the skin.

EP 564 307 A1 describes a formulation which buffers the pH in body fluids, in particular urine, to approx. 5 when the fluid comes into contact with the formulation. The aim is for the urine to have an acid pH if it leaks out onto the skin since weakly acid urine is less harmful to the skin. It is emphasized, however, that the invention does not affect the pH of the skin; instead, it simply adjusts the pH of the leaking fluid.

EP 276 561 A1 describes a self-adhesive tape which has an analgesic and anti-inflammatory effect. The pH of the adhesive is within the range 4-9. No buffer is added and the function is not to adjust the pH of the skin via the adhesive, either.

JP 61282313 describes a self-adhering adhesive which contains proteolytic enzymes. Following application, these enzymes are intended to leak out onto the skin and to dissolve the keratin on the skin in order, in contrast to our invention, to impair the barrier effect of the skin. A pH modifier, which has the optimum pH for the enzyme in question, is also added. The modifier leaks out together with the enzyme.

The aim of the present invention is to produce a skin-friendly adhesive which ensures an appropriate pH on skin which is covered with such an adhesive.

### SUMMARY OF THE INVENTION

According to the invention, this aim is achieved by means of a skin-friendly adhesive according to claim 1.

In a preferred embodiment, the pH is lowered to between 3.5-6.0, preferably between 4.5-5.8, and, more preferably, between 4.9-5.5.

The buffer consists of alpha- or beta-hydroxyacids and their salts, for example sodium citrate and citric acid or sodium propionate and propionic acid.

The buffer can also consist of an inorganic buffer, for example a phosphate buffer consisting of NaH₂PO₄ and Na₂HPO₄.

The pH-lowering substance can also consist of an ampholytic substance whose carboxyl groups are partially substituted by a positive ion, preferably Na⁺.

Alternatively, the pH-lowering substance can consist of a mixture of one or more components consisting of alpha- or beta-hydroxyacids and their salts, such as sodium citrate and citric acid and sodium propionate and propionic acid, phosphate buffer, such as NaH₂PO₄ and Na₂HPO₄, and an ampholytic substance whose carboxyl groups are partially substituted by a positive ion, preferably Na⁺.

In addition to containing a substance which lowers the pH of the skin, the skin-friendly adhesive according to the invention can also contain an additive consisting of a preparation having a pharmaceutical effect.

In addition, the adhesive should adhere to the skin with a force of between 0.5-3 N, preferably 0.5-2 N and most preferably 0.7-1.5 N.

The invention also relates to a wound dressing which comprises an adhesive-coated supporting material which is coated with an adhesive as described above. In this connection, a wound dressing is also understood as meaning a sticking plaster and a plaster for treating skin diseases. The supporting material preferably consists of a flexible flat material, such as a plastic film, a knitted or woven fabric, a foam lamina or a nonwoven fabric. The supporting material is adhesive-coated either on one side or on both sides. In a variant, parts of the supporting material can lack an adhesive coating.

The invention also relates to the use of an adhesive as described above on a sanitary towel, a panty liner, a wig, an ostomy bag or ostomy compress, an explant or false eyelashes.

### BRIEF DESCRIPTION OF THE FIGURES

The invention will now be described with reference to the attached Figures, of which;
Fig. 1 shows a diagram of a sticking plaster in accordance with one embodiment of the invention,
Fig. 2 shows a diagram of a wound dressing in accordance with another embodiment of the invention, and
Fig. 3 diagrammatically illustrates a method for determining the adhesion of a dressing to the skin.

### DESCRIPTION OF EMBODIMENTS

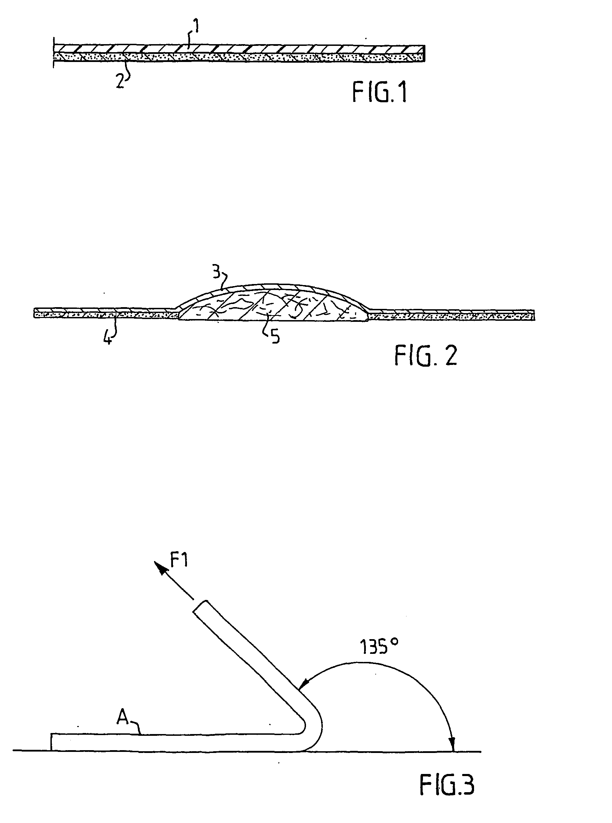

Figure 1 shows a diagram of a sticking plaster which consists of a supporting material 1 which is coated, on one of its sides, with an adhesive layer 2. The adhesive layer 2 consists of an adhesive matrix to which is added a substance which, on contact with skin moisture, lowers the pH of the skin to an appropriate value if this pH is too high.

The supporting material 1 consists of an easily flexed material to which the adhesive readily adheres, for example a textile material, a polymer foam, a plastic material or a nonwoven material. The supporting material should consist of a flat ("unidimensional") material, for example a plastic film, a knitted or woven fabric or a foam lamina. While the adhesive coating can be restricted to being only present on one side of the supporting material, it can also constitute an impregnation in porous supporting materials, with the adhesive penetrating into the supporting material and reaching out onto its other side. The adhesive coating can also surround the supporting material entirely, such that broadly speaking all the parts of the supporting material are surrounded by adhesive. The adhesive does not need to cover the whole of the supporting material but can instead be restricted to a part of the supporting material. The invention primarily relates to those self-adhering adhesives whose tackiness is not dependent on a particular water content, such as many self-adhesive hydrogels which contain large proportions of water.

The self-adhering adhesive in the adhesive layer 2 is of the dry self-adhering adhesive type which, by and large, does not contain water and which does not need to contain water in order to be self-adhering. However, small residues of water may be present due to incomplete drying or as a result of the equilibrium with the natural atmospheric moisture. The adhesive can consist of different skin-friendly adhesive types which are generally used for dressings and other products which are to be attached to the skin, such as solvent-based acrylate adhesives, water emulsion-based acrylate adhesives, self-adhering hot-melt adhesives, self-adhering silicone adhesives, etc. Mixtures of different adhesive types can also be used. In this present context, self-adhering is understood as meaning an adhesive which attaches the dressing/plaster to normal, non-wet skin by means of adhesion and that the dressing does not fall off due to its own weight. The dressing should be able to be loaded by the force of gravity for at least one hour and at the same time cope with body movements which are not too violent.

The force with which the dressing adheres to the skin is measured using the following method, which was developed by the inventor and which is illustrated diagrammatically in Figure 3. Strips A of the dressing/plaster having a width of 25 mm are applied to the backs of eight individuals and allowed to remain in place for four hours. After that, the strips A are peeled off at a speed of 25 mm/s and the peeling force F1 is measured. The angle of peeling, i.e. the obtuse angle which is formed, on peeling, between the skin and the peeled-off part of the strip A, should be 135°, as shown in Figure 3. The mean value of the force F1 should be 0.2-3 N for a correctly functioning dressing or correctly functioning plaster. It has been found that very good function is obtained when the force F1 is within the interval 0.5-2 N, preferably 0.7-1.5 N.

In a 180° peel adhesion test in accordance with ASTM-3330 M-81, for measuring the force of adhesion to polished steel plates, the force which is measured should be between 0.3-2.0 N, expediently 0.5-1.5 N and preferably 0.7-1.0 N.

The adhesive in the adhesive layer 2 has been provided with an additive of acidic substances which imparts a weakly acid pH to the adhesive surface when the adhesive comes into contact with skin moisture. This pH-lowering substance is uniformly distributed in the adhesive layer (or, if the adhesive layer is thick, in at least the outermost part of the adhesive layer which is in contact with the skin) in order to achieve the most uniform effect. When skin moisture comes into contact with the acid substance, which is located in the dry state in the adhesive, some of the molecules pass into solution and bring about the desired pH in the moisture layer of the skin. In order to ensure that the pH of the skin comes to lie within the desired range, it is expedient to select those additives which have a buffering effect within the correct pH interval. Alternatively, additives are selected which are so weakly acid, even in the concentrated state, that they are unable to lower the pH below the desired interval.

If it is decided to use a buffer as the acidic additive, this buffer can then be a two-component buffer consisting of organic acids and their sodium salts, for example alpha- or beta-hydroxyacids and their salts, such as sodium citrate and citric acid or sodium propionate and propionic acid. Buffers based on the sodium salts of phosphoric acid can also be used. Examples of this type of two-component buffer are ascorbic acid/sodium ascorbate, citric acid/disodium hydrogen phosphate, citric acid/sodium citrate, glycolic acid/sodium glycolate, lactic acid/sodium lactate, malonic acid/sodium malonate, tartaric acid/sodium tartrate, and salicylic acid/sodium salicylate. The abovementioned buffer components can also be combined in many different ways and consist of three or more buffer components. It is also possible to use those substances, as the buffer, which possess both acidic and basic groups within the same molecule and which thereby have a buffering effect within themselves. For example, polymers possessing carboxyl groups of which some are in acid form and others in base form (-COOH and -COO⁻Na⁺, respectively) can have a buffering effect within the correct range. An example of polymers of this type is carboxymethyl cellulose some of whose carboxyl groups consist of the sodium salt of the carboxylic acid. Pectin also has a buffering effect within the correct range. The buffer system can also consist of mixtures of the abovementioned buffers. Furthermore, the acidic substances are expediently distributed homogeneously in the adhesive. However, in the case of thick adhesive layers, for example those which extend on both sides of the supporting material, it can be sufficient to admix the acidic substances with that part of the adhesive layer which is adjacent to the skin during use.

The invention which is described below solves the problem of the pH being too high at the interface between skin and adhesive under self-adhesive wound dressings and sticking plasters. A self-adhesive dressing or a plaster in accordance with the invention is provided with a dry self-adhering adhesive which is applied to the dressing. The self-adhering adhesives to which this present document relates normally bind only extremely small quantities of skin moisture. Transport of skin moisture in the adhesive layer is therefore highly restricted. The buffer particles are frequently well-protected by the adhesive, something which makes it appreciably more difficult for the skin moisture to activate the pH-lowering substances. In order to obtain an adequate effect despite this, it is necessary to use a large excess of acidic substances. The quantity of acid or buffer which is required can be reduced by adding hydrophilic polymers to the adhesive. These hydrophilic polymers are expediently of the hydrogel type which, on contact with moisture, assimilates moisture by means of swelling absorption. When the hydrogel molecules, which consist of long polymer chains, swell, they open up channels in the adhesive thereby allowing the skin moisture to come into contact with a larger proportion of the acidic additive. This makes it possible to increase the supply of acidic substances and increase the period during which the pH is lowered. In order to achieve the greatest and most uniform effect, these hydrophilic polymers should be well distributed in the adhesive. It is also possible to select those additives which combine all the abovementioned functions; 1) a weakly acid pH with 2) a certain degree of buffering capacity and 3) swelling absorption on the addition of water, for example the sodium salt of carboxymethyl cellulose or pectin.

A hydrogel which is to be used in a dressing in accordance with the invention should be non-toxic and tissue-friendly. Such hydrogels are found among the hydrogels which are used as foodstuff thickeners. Examples are carboxymethyl cellulose and its sodium salts, polyvinylpyrrolidone, xanthan gum, pectin, various starch derivatives, etc. Synthetic hydrogels of the polyacrylate type or polyurethane type can also be suitable for the purpose. Superabsorbents of the type used in nappies can also be employed.

When the adhesive-coated surface of the dressing is moistened with the smallest quantity of water which is required for measuring the pH, it should be possible to measure a pH within the range 3.5-6.0 within a few hours. Preferably, the pH is within the range 4.5-5.8 and most preferably within the range 4.9-5.5.

When a dressing or plaster according to the invention is used on skin which has an elevated pH (>6.0) due to external circumstances or disease, it is possible to measure a lowering of the pH within the interval 4.0-6.0 after a few hours of application when using current methods for measuring skin pH (references) or when using the operating instructions for the PH 900^{®} SKIN pH meter, CK electronic GmbH, 50829 Cologne/Germany. When a dressing or plaster in accordance with the invention is used on skin which has a normal pH, a pH within this interval is then maintained, in contrast to what would have been the case if the dressing had lacked the acidic additives.

The present invention makes it possible to control the pH at the interface between adhesive and skin by optimizing the buffer capacity and the rate of release of the buffer. The composition of the buffer system is expediently selected such that the adhesive provides the desired pH on dry skin, where the adhesive is only loaded with very small quantities of skin moisture, without having very much effect on the pH deep down in a discharging wound which is covered by the dressing, due to the fact that the buffer is greatly diluted at this location. It is important that, while the buffering effect is sufficiently large to ensure the desired pH on the skin during the period the dressing/plaster is used, this effect is also sufficiently small for it not to affect the pH in the moist, exudating wound more than marginally.

Preparations having a pharmaceutical effect on the skin or wound can also be admixed with the adhesive layer.

Figure 2 shows a diagram of a dressing in accordance with a second embodiment of the invention. This dressing also comprises a supporting material 3 which is provided, outside of a central region, with an adhesive layer 4 in which is present a substance which, on contact with skin moisture, lowers the pH of the skin to a suitable value if this pH is too high. An absorbent pad 5 is arranged in the central region of the dressing. This pad can consist of a foam pad which, on its surface of contact with a wound, is covered with a thin silicone gel layer which prevents the pad adhering to the surface of the wound. Such a pad is disclosed by US-A-6,051,747. The supporting materials, adhesives and substances which are suitable for the plaster described in connection with Figure 1 are also suitable for the dressing described in Figure 2.

Adhesive in accordance with the invention can be used on different types of self-adhesive dressings and skin plasters. Examples are self-adhesive plasters having an absorbent wound pad of the Band-aid (Johnson & Johnson), Leukoplast (Beiersdorf), Salvekvick (Cederrot), Airstrip (Smith & Nephew), Mepore (Mölnlycke), Tielle (Johnson & Johnson) and Allevyn Adhesive (Smith & Nephew) type. Another suitable product type is represented by sticking plasters and tapes for use on the skin, of the Mefix (Mölnlycke), Steri-Strip (3M), Micropore (3M), Leukosilk (Beiersdorf), Mepiform (Mölnlycke) and Cicacare (Smith & Nephew) type. Occlusive self-adhesive wound dressings of the Duoderm (Convatec), Op-Site (Smith & Nephew) and Mitraflex (Mölnlycke) type are a dressing type which is suitable for the invention. The abovementioned product types are all coated with adhesive on one of their sides. The invention can also be applied to a product type where a supporting material is entirely impregnated with adhesive, for example Mepitel (Mölnlycke), which consists of a perforated textile supporting material which is entirely surrounded by a soft, self-adhesive silicone gel (US-A-4,921,704).

Furthermore, a plaster in accordance with the invention can be used for treating skin diseases. It is also possible to affix an adhesive in accordance with the invention to a supporting material for some other product which is to be attached to the skin, such as sanitary towels, wigs, ostomy bags, ostomy compresses, explants, false eyelashes, etc.

### Implementation examples

1. 40% ammonia was added, while stirring, to an aqueous acrylate adhesive of the dispersion type (RHODOTAC 315, RHODIA Limited, Manchester, UK) until a pH of 5 was reached. After that, sodium carboxymethyl cellulose (CMC: Carboxymethylcellulose Natriumsalz [carboxymethyl cellulose sodium salt], Art. 3333.1, Carl Roth GmbH, Karlsruhe, Germany) was added slowly while stirring vigorously. 1.5 ml of a pH 5.0 buffer consisting of NaH₂PO₄°2H₂O + citric acid (in the ratio by weight of 66.3% : 33.7%) were added slowly to 20 g of the dispersion while stirring. While the concentrations of this buffer were different in the different experiments, the weight ratio between NaH₂PO₄·2H₂O and citric acid was constant. The adhesive was streaked out in a thin layer (approx. 40 g/m²) on a plastic-coated paper and dried at 70°C. 0.1 ml of water was deposited in a drop on the adhesive surface and the pH on the adhesive surface was measured with a flat pH electrode after 5 minutes. Different compositions A-H were tested and were found to lower the pH to different extents as compared with the pure adhesive. The quantity of CMC and the quantity of buffer are given in % of the dry weight of the final adhesive mixture.

| | CMC (% dry weight) | Buffer (% dry weight) | PH |
|---|---|---|---|
| A | 0 | 0 | 7.1 |
| B | 2 | 0 | 6.0 |
| C | 5 | 0 | 5.1 |
| D | 10 | 0 | 4.8 |
| E | 0 | 10 | 5.6 |
| F | 2 | 10 | 5.2 |
| G | 5 | 10 | 5.2 |
| H | 10 | 10 | 4.9 |

2. 40% ammonia was added, while stirring, to an aqueous acrylate adhesive of the dispersion type (RHODOTAC 315, RHODIA Limited, Manchester, UK) until a pH of 5 was reached. After that, different types of foodstuff thickener (Pectin, citrus, art. No. 1.3651-1, KEBO LabAB, Stockholm, Sweden and XanthanGummi [xanthan gum], art. No. 28,602-8, Aldrich-Chemie, Steinheim, Germany) were added slowly while stirring vigorously. 1.5 ml of a pH 5.0 buffer consisting of NaH₂PO₄·2H₂O + citric acid (in the ratio by weight of 66.3% : 33.7%) were added slowly to 20 g of the dispersion while stirring. While different concentrations of this buffer were used in the different experiments, the ratio between the NaH₂PO₄·2H₂O and the citric acid was constant. The adhesive was streaked out in a thin layer (approx. 40 g/m²) on a plastic-coated paper and dried at 70°C. 0.1 ml of water was deposited in a drop on the adhesive surface. The sample was covered with plastic in order to protect it from drying out. The pH on the adhesive surface was measured with a flat pH electrode after 120 minutes. Different compositions A-F were tested and were found to lower the pH to different extents as compared with pure adhesive. The quantity of CMC and the quantity of buffer are given in % of the dry weight of the final adhesive mixture.

| | Pectin (% of dry weight) | Xanthan gum (% of dry weight) | Buffer (% of dry weight) | PH |
|---|---|---|---|---|
| A | 0 | 0 | 0 | 6.8 |
| B | 0 | 0 | 5 | 4.9 |
| C | 2 | 0 | 0 | 4.7 |
| D | 2 | 0 | 5 | 5.3 |
| E | 0 | 2 | 0 | 5.7 |
| F | 0 | 2 | 5 | 5.3 |

3. The two components A and B, respectively, in a two-component silicone adhesive system (Wacker Silicon 45554, Wacker-Chemie GmbH, Munich, Germany) were mixed in equal parts. Finely divided pectin (the same as in example 2) and pH 4.5 buffer consisting of NaH₂PO₄·2H₂O + citric acid (in the weight ratio of 60.7% : 39.3%) were added slowly, while stirring, in different quantities to give final concentrations as shown in the table below. The mixture was streaked out on a teflonized cloth in a thin approx. 1 mm layer. A nonwoven material was laid on top of the silicone as the supporting material and the sample was placed in a 130°C oven for 5 minutes in order to cure the silicone. On curing, the cured silicone forms a soft self-adhering, gelatinous adhesive which attaches to the skin. After cooling, the silicone was peeled off the teflon surface together with the nonwoven supporting material. A moistened filter paper was laid on that side of the silicone surface which had been in contact with the teflon during the curing. The filter paper was covered in order to protect it from drying out. After one hour, the pH was measured with a flat pH electrode. Different compositions were found to lower the pH to varying extents, as shown in the following table.

| | Pectin (%) | Buffer (%) | PH |
|---|---|---|---|
| A | 0 | 0 | 7.9 |
| B | 1 | 0 | 5.8 |
| C | 10 | 0 | 4.6 |
| D | 0 | 1 | 5.4 |

4. A soft, self-adhering hot-melt adhesive, Dispomelt 70-4647 from National, Bridgewater, New Jersey, USA, was melted at 150°C. Pectin (the same quality as in example 2) and citric acid/phosphate buffer (the same buffer as in example 1), respectively, were added slowly to the heated hot-melt adhesive while stirring vigorously. The different samples were transferred to a heated teflon sheet (150°C) and streaked out in a uniform layer of 1 mm in thickness. A supporting material consisting of fibre cloth was laid on top and allowed to stick to the upper surface of the adhesive layer. The function of the fibre cloth was to reinforce the sample so that it was possible to peel it off the teflon surface without it coming apart. The samples were left to cool so that they' would congeal. After cooling, the samples were peeled off the teflon surface together with the fibre cloth. The congealed adhesive was soft and self-adhering and could be affixed to skin. A moistened filter paper was laid on the lower side of the sample, i.e. that side of the sample which had been in contact with the teflon surface during curing. The filter paper was covered over in order to protect it from drying out. After an hour, the pH was measured with a flat pH electrode.

Some samples in which pectin was admixed were prepared without any fibre cloth on the upper side. The pH was measured on these samples in the same way as described above except that the measurement was made on the upper side of the sample. The different samples were found to lower the pH to varying extents as shown in the following table.

| | PH |
|---|---|
| Hot-melt adhesive without additive (lower side) | 6.3 |
| Hot-melt adhesive containing 10% by weight pectin (lower side) | 5.2 |
| Hot-melt adhesive containing 10% by weight pectin (upper side) | 4.7 |
| Hot-melt adhesive containing 10% by weight citric acid/phosphate buffer, pH 5.0 (lower side) | 5.2 |

A PH900 Skin pH meter, Courage+Khazaka, Cologne, Germany, was used for measuring the pH in the above-described implementation examples.

The experiments which were carried out demonstrate that there is a good possibility of influencing the pH in the contact surface between the skin and the adhesive. Experiments also show that there are many parameters which affect the quantity of additive which is required (adhesive type, buffer type, hydrogel type, proportions between adhesive, buffer and hydrogel, etc.). It is very likely that optimizations would make it possible to substantially decrease the quantities of additive employed.

## Claims

1. Dry, self-adhesive and skin-friendly adhesive, which does not contain water and which does not need to contain water in order to be self-adhering, **characterized in that** it contains a substance which, on contact with pH-neutral water, lowers the pH of the water chosen from the group of
alpha- or beta-hydroxyacids and their salts,
sodium citrate and citric acid,
sodium propionate and propionic acid,
an inorganic buffer, for example a phosphate buffer,
NaH₂PO₄ and Na₂HPO₄,
of an ampholytic substance some of whose carboxyl groups are substituted by a positive ion, preferably Na⁺, and
a mixture of one or more components consisting of alpha- or beta-hydroxyacids and their salts, such as sodium citrate and citric acid and sodium propionate and propionic acid, phosphate buffer, such as NaH₂PO₄ and Na₂HPO₄, and an ampholytic substance, some of whose carboxyl groups are substituted by a positive ion, preferably Na⁺, **in that** hydrophilic polymers of the hydrogel type which, on contact with moisture, assimilates moisture by swelling absorption are added to the adhesive, and **in that** it consists of a solvent-based or water emulsion-based acrylate adhesive, a tacky hot-melt adhesive, or a silicone adhesive which adheres to skin, or a mixture of two or more of the said adhesive types.

2. Adhesive according to Claim 1, **characterized in that** the pH is lowered to between 3.5-6.0, preferably between 4.5-5.8 and and more more preferably between 4.9-5.5.

3. Adhesive according to Claim 1 or 2, **characterized in that** it contains an additive consisting of a preparation having a pharmaceutical effect.

4. Adhesive according to any one of the preceding claims, **characterized in that** the adhesive adheres to skin with a force of between 0.2-3 N, preferably 0.5-2 N and most preferably 0.7-1.5 N.

5. Wound dressing comprising an adhesive-coated supporting material (1;3), **characterized in that** the supporting material (1;3) is coated with an adhesive (2;4) in accordance with any one of Claims 1-4.

6. Wound dressing according to Claim 5, **characterized in that** the supporting material (1;3) consists of a flexible flat material, such as a plastic film, a knitted or woven fabric, a foam lamina or an unwoven fibre fabric.

7. Wound dressing according to Claim 6, **characterized in that** the supporting material is adhesive-coated on both sides.

8. Wound dressing according to Claim 5, 6 or 7, **characterized in that** parts of the supporting material (3) lack an adhesive coating.

9. Wound dressing according to any one of Claims 5-8, **characterized in that** the adhesive consists of a solvent-based or water emulsion-based acrylate adhesive, a tacky hot melt adhesive, or a silicone adhesive which adheres to skin, or a mixture of two or more of the said adhesive types.

10. Use of an adhesive according to any one of Claims 1-4 on a sanitary towel, a panty liner, a wig, an ostomy bag or ostomy compress, an explant or false eyelashes.

## Patentansprüche

1. Trockenes, selbstklebendes und hautfreundliches Klebemittel, das kein Wasser enthält und das kein Wasser erfordert, um selbstklebend zu sein, **dadurch gekennzeichnet, dass** es eine Substanz enthält, die bei Kontakt mit pH-neutralem Wasser den pH-Wert des Wassers erniedrigt, ausgewählt aus der Gruppe von α-oder β-Hydroxysäuren und ihren Salzen, Natriumcitrat und Zitronensäure, Natriumpropionat und Propionsäure, anorganischen Puffern, z.B. Phosphatpuffer, NaH₂PO₄ und Na₂HPO₄, ampholytischen Substanzen, bei denen einige von deren Carboxylgruppen mit einem positiven Ion, vorzugsweise Na⁺, substituiert sind, und Mischungen von einer oder mehreren Komponenten, bestehend aus α- oder β-Hydroxysäuren und ihren Salzen, wie z.B. Natriumcitrat und Zitronensäure und Natriumpropionat und Propionsäure, Phosphatpuffern, wie z.B. NaH₂PO₄ und Na₂HPO₄, und einer ampholytischen Substanz, wobei einige von deren Carboxylgruppen mit einem positiven Ion, vorzugsweise mit Na⁺, substituiert sind, **dadurch**, dass hydrophile Polymere vom Hydrogeltyp zu dem Klebemittel zugegeben sind, die bei Kontakt mit Feuchtigkeit durch Quellungsabsorption Feuchtigkeit aufnehmen,
und **dadurch**, dass es aus einem auf Lösungsmittel basierenden oder auf einer Wasseremulsion basierenden Acrylat-Klebemittel, einem klebrigen Heissschmelzklebemittel oder einem Silicon-Klebemittel, das an die Haut anhaftet, oder einer
Mischung von zwei oder mehreren der Klebemitteltypen besteht.

2. Klebemittel gemäss Anspruch 1, **dadurch gekennzeichnet, dass** der pH-Wert auf zwischen 3,5 und 6,0, vorzugsweise zwischen 4,5 und 5,8 und stärker bevorzugt zwischen 4,9 und 5,5, verringert wird.

3. Klebemittel gemäss Anspruch 1 oder 2, **dadurch gekennzeichnet, dass** es ein Additiv enthält, das aus einer Zubereitung besteht, die eine pharmazeutische Wirkung aufweist.

4. Klebemittel gemäss irgendeinem der vorhergehenden Ansprüche, **dadurch gekennzeichnet, dass** das Klebemittel mit einer Kraft zwischen 0,2 und 3 N, vorzugsweise 0,5 und 2 N und am stärksten bevorzugt 0,7 und 1,5 N, an die Haut anhaftet.

5. Wundverband, umfassend ein mit einem Klebemittel beschichtetes Trägermaterial (1; 3), **dadurch gekennzeichnet, dass** das Trägermaterial (1; 3) mit einem Klebemittel (2; 4) gemäss irgendeinem der Ansprüche 1 bis 4 beschichtet ist.

6. Wundverband gemäss Anspruch 5, **dadurch gekennzeichnet, dass** das Trägermaterial (1; 3) aus einem flexiblen flachen Material, wie z.B. einer Kunststoffolie, einem gewirkten oder gewebten Stoff, einer Schaumschicht oder einem ungewebten Faserstoff, besteht.

7. Wundverband gemäss Anspruch 6, **dadurch gekennzeichnet, dass** das Trägermaterial auf beiden Seiten mit Klebemittel beschichtet ist.

8. Wundverband gemäss Anspruch 5, 6 oder 7, **dadurch gekennzeichnet, dass** Teile des Trägermaterials (3) keine Klebemittelbeschichtung aufweisen.

9. Wundverband gemäss irgendeinem der Ansprüche 5 bis 8, **dadurch gekennzeichnet, dass** das Klebemittel aus einem auf Lösungsmittel basierenden oder auf einer Wasseremulsion basierenden Acrylat-Klebemittel, einem klebrigen Heissschmelzklebemittel oder einem Silicon-Klebemittel, das an die Haut anhaftet, oder einer Mischung von zwei oder mehreren dieser Klebemitteltypen besteht.

10. Verwendung eines Klebemittels gemäss irgendeinem der Ansprüche 1 bis 4 auf einer Binde, einer Slipeinlage, einer Perücke, einer Tüte für die Darmöffnung oder einem Verband für die Darmöffnung, einem Explantat oder falschen Wimpern.

## Revendications

1. Adhésif sec, auto-adhésif et respectueux de la peau qui ne contient pas de l'eau et qui n'a pas besoin de contenir de l'eau afin d'être auto-adhésif, **caractérisé en ce qu'**il contient une substance qui, en contact avec de l'eau à pH neutre, diminue le pH de l'eau, et choisie parmi le groupe constitué
des alpha-hydroxyacides ou des bêta-hydroxyacides et de leurs sels,
du citrate de sodium et de l'acide citrique,
du propionate de sodium et de l'acide propionique,
d'un tampon minéral, par exemple un tampon de phosphate,
de NaH₂PO₄ et de Na₂HPO₄,
d'une substance ampholytique dont certains groupes carboxyle sont substitués par un ion positif, de préférence Na⁺, et
d'un mélange d'un ou de plusieurs composants constitués des alpha-hydroxyacides et des bêta-hydroxyacides et de leurs sels, tels que du citrate de sodium et de l'acide citrique, du propionate de sodium et de l'acide propionique, un tampon de phosphate, tel que du NaH₂PO₄ et du Na₂HPO₄, et d'une substance ampholytique, dont certains groupes carboxyle sont substitués par un ion positif, de préférence Na⁺,
**en ce que** des polymères hydrophiles du type hydrogel qui, en contact avec de l'humidité, assimilent l'humidité par absorption par gonflement, sont ajoutés à l'adhésif, et **en ce que** l'adhésif se compose d'un adhésif acrylate à base d'émulsion à l'eau ou à base de solvant, d'un adhésif collant thermofusible, ou d'un adhésif au silicone qui adhère à la peau, ou d'un mélange de deux ou plus desdits types d'adhésif.

2. Adhésif selon la revendication 1, **caractérisé en ce que** le pH est abaissé entre 3,5 et 6,0, de préférence entre 4,5 et 5,8 et de manière plus préférée entre 4,9 et 5,5.

3. Adhésif selon la revendication 1 ou 2, **caractérisé en ce qu'**il contient un additif constitué d'une préparation ayant un effet pharmaceutique.

4. Adhésif selon l'une quelconque des revendications précédentes, **caractérisé en ce que** l'adhésif adhère à la peau avec une force entre 0,2 et 3 N, de préférence entre 0,5 et 2 N et de manière plus préférée entre 0,7 et 1,5 N.

5. Pansement comprenant un matériau de support revêtu d'adhésif (1 ; 3), **caractérisé en ce que** le matériau de support (1 ; 3) est revêtu d'un adhésif (2 ; 4) conformément à l'une quelconque des revendications 1 à 4.

6. Pansement selon la revendication 5, **caractérisé en ce que** le matériau de support (1 ; 3) se compose d'un matériau plat flexible, tel qu'un film plastique, un tissu tricoté ou tissé, une lame en mousse ou un tissu en fibres non tissé.

7. Pansement selon la revendication 6, **caractérisé en ce que** le matériau de support est revêtu d'adhésif des deux côtés.

8. Pansement selon la revendication 5, 6 ou 7, **caractérisé en ce que** des parties du matériau de support (3) ne présentent pas de revêtement adhésif.

9. Pansement selon l'une quelconque des revendications 5 à 8, **caractérisé en ce que** l'adhésif se compose d'un adhésif acrylate à base d'émulsion à l'eau ou à base de solvant, d'un adhésif collant thermofusible, ou d'un adhésif au silicone qui adhère à la peau, ou d'un mélange de deux ou plus desdits types d'adhésif.

10. Utilisation d'un adhésif selon l'une quelconque des revendications 1 à 4 sur une serviette hygiénique, un protège-slip, une perruque, une poche de stomie ou une compresse de stomie, un explant ou des faux cils.
